Europäisches Patentamt

(19) European Patent Office

Office européen des brevets

(11) Publication number: **0 320 752 B1**

# EUROPEAN PATENT SPECIFICATION

(12)

(45) Date of publication of patent specification: **24.06.92** �serialint. Cl.⁵: **G01N 33/53**, B01L 3/14

(21) Application number: **88120324.4**

(22) Date of filing: **06.12.88**

(54) **Analytical reagent mixing apparatus for performing sequential analytical reactions.**

(30) Priority: **17.12.87 US 133997**

(43) Date of publication of application:
**21.06.89 Bulletin 89/25**

(45) Publication of the grant of the patent:
**24.06.92 Bulletin 92/26**

(84) Designated Contracting States:
**AT BE CH DE ES FR GB IT LI LU NL SE**

(56) References cited:
**EP-A- 0 204 579        EP-A- 0 213 653
AU-A- 540 523          DE-A- 3 403 264
US-A- 3 783 105        US-A- 4 239 746**

(73) Proprietor: **MILES INC.**
**1127 Myrtle Street
Elkhart,IN 46515(US)**

(72) Inventor: **Kheiri, Mohammed A.
59092 Winding Waters Ln.
Elkhart, IN 46514(US)**
Inventor: **Mammolenti, Joseph
12060 Buttercup Circle
Granger, IN 46530(US)**
Inventor: **Morris, David L.
12174 Ronda Drive
Elkhart, IN 46514(US)**
Inventor: **Siddons, George V.
428 W. Beardsley Avenue
Elkhart, IN 46514(US)**
Inventor: **Sommer, Ronald G.
55745 Merle St.
Elkhart, IN 46514(US)**

(74) Representative: **Kirchner, Dietrich, Dr. et al
c/o BAYER AG Konzernverwaltung RP Paten-
tabteilung
W-5090 Leverkusen, Bayerwerk(DE)**

**Description**

BACKGROUND OF THE INVENTION

The present invention relates to analytical reactions for detecting a determinable substance in a liquid test sample. In particular, the present invention relates to the determination of an analyte in a liquid test sample involving reactions among the analyte and analytical reagents which require various manipulative steps, such as periods of incubation and mixing, to make such determination.

Various analytical procedures have evolved from the need to determine the amount of analyte in a liquid test sample. In particular, various clinical assays have been developed to monitor, for example, therapeutic drug levels in biological fluids from patients being treated with such drugs for various illnesses and diseases. Typically, such clinical assays are employed to assess toxicity, inadequacy of the therapeutic response, and to enable dose adjustment until an adequate response is achieved, particularly where there is considerable overlap between toxic and therapeutic levels for a particular drug.

However, such analytical procedures and clinical assays often require complicated, expensive instruments and trained, experienced technicians for their performance. Furthermore, where such assays do not necessarily require the use of such complicated instruments, such assays and procedures nevertheless often require the performance of sequential reactions. Due to the sequential nature of the assay protocol, a number of time consuming and sequenced manipulative steps, such as pipetting, periods of incubation, separation steps, and the like, are required which are time consuming and subject to intolerable errors that can lead to inaccurate results.

Accordingly, it is an object of the present invention to provide a convenient method and apparatus for performing sequential analytical reactions which requires a minimal number of manipulative steps.

Another object of the present invention is to provide an inexpensive apparatus for performing sequential analytical reactions which is easy to operate.

Further, it is an object of the present invention to provide a method and apparatus for performing sequential analytical reactions for the accurate determination of an analyte from a liquid test sample.

SUMMARY OF THE INVENTION

The present invention provides an analytical reagent mixing apparatus for performing sequential analytical reactions, such as immunoassays, whereby a detectable signal is provided which is measured and correlated to the amount of an analyte present in a liquid test sample. Typically, such analytical reactions involve analytical reagents which are sequentially combined with a liquid test sample, and require a number of incubation, pipetting and mixing steps in order to make such determination. For example, where two analytical reagents are involved, it is often necessary to combine, mix, and incubate one of the reagents with the liquid test sample and then add the other reagent thereto, followed by additional mixing and incubation steps thereof.

According to the present invention, the analytical reagent mixing apparatus automatically performs such mixing and incubation steps to provide a simple, convenient, and time efficient method for carrying out such analytical reactions. In particular, the analytical reagent mixing apparatus comprises an analytical reagent assembly comprising first and second open receptacles having first and second analytical reagents disposed therein and a lid member engaged with the receptacles in a fluid-tight manner, and means for rotating the analytical reagent assembly. The lid member includes means for providing open liquid communication between the first and second receptacles, such as a common chamber or channel, to facilitate the transfer and mixing of the first and second analytical reagents between the first and second receptacles. The means for rotating the analytical reagent assembly is capable of rotating the analytical reagent assembly, preferably in alternating clockwise and counterclockwise directions, to manipulate the analytical reagents whereby depending upon the degree of rotation of the analytical reagent assembly, the analytical reagents either remain and are mixed separately in their respective receptacles, or are caused to flow through the means for providing open liquid communication and thereby transferred and mixed between the receptacles. In particular, such manipulation of the analytical reagents is the result of rotating the analytical reagent assembly in a plane defined by the longitudinal axes of the reagent receptacles and about an axis of rotation defined by a point which intersects the plane. For example, when it is desired to maintain and mix separately the analytical reagents in their respective receptacles, the analytical reagent assembly is rotated about the axis of rotation at less than about 90° relative to vertical in order to prevent the analytical reagents from flowing between the receptacles through the lid member. Alternatively, when it is desired to mix the analytical reagents with each other, the analytical reagent assembly is rotated about the axis of rotation greater than about 90° relative to vertical or, more preferably, rotated a complete revolution or series of revolutions (360°) about the axis of rotation, whereby the analytical reagents are caused to flow through the lid member and mix with each other between the receptacles.

It is to be understood that according to the present invention, the lid member provides means to transfer and mix the analytical reagents between the reagent receptacles and thereby eliminates the cumbersome manual pipetting steps otherwise necessary to transfer and mix the analytical reagents. Accordingly, the manipulation of the analytical reagents is intended to include any movement of the analytical reagent assembly which maintains and mixes the analytical reagents separately in their respective receptacles and, when desired, causes the analytical reagents to flow through the means for providing open communication.

The analytical reagent mixing apparatus further comprises timing means, such as a microprocessor or a computer, programmed to control the number, direction and periods of time of such rotating movement, as well as periods of stationary incubation positions. Preferably, the analytical reagent assembly, the means for rotating the analytical reagent assembly, and the timing means are encased in a housing within which the analytical reactions can be performed.

In carrying out the method of the present invention, a first liquid reaction mixture is formed in the first receptacle by adding the liquid test sample to the first analytical reagent, and the lid member is engaged with the receptacles in a fluid-tight manner to provide the analytical reagent assembly of the present invention. The assembly is then rotated or otherwise manipulated for a predetermined period of time, whereby the first liquid reaction mixture is maintained and mixed separately in the first receptacle, and then the assembly is rotated or otherwise manipulated for a predetermined period of time, whereby the first liquid reaction mixture and the second analytical reagent traverse the lid member through the means for providing open liquid communication and are thereby mixed with each other between the receptacles to form a second liquid reaction mixture thereof. After a predetermined period of time, the detectable signal provided by the second liquid reaction mixture, or an aliquot removed therefrom, is measured and correlated to the amount of analyte present in the liquid test sample.

## BRIEF DESCRIPTION OF THE DRAWINGS

Fig. 1 is an exploded front view of the analytical reagent assembly.

Fig. 2 is an exploded side view of the analytical reagent assembly taken along line 2-2 of Fig. 1.

Fig. 3 is a cross-sectional front view of the analytical reagent assembly.

Fig. 4 is a bottom view of the analytical reagent assembly taken along line 4-4 of Fig. 1.

Fig. 5 is a top view of the analytical reagent assembly taken along line 5-5 of Fig. 1.

Fig. 6 is a partial perspective view of the proximal end of a tray for holding one or more of the analytical reagent assembly.

Fig. 7 is a perspective view of the analytical reagent mixing apparatus encased in a housing.

Fig. 8 is a cross-sectional side view of a filtration device partially inserted into an open receptacle of the analytical reagent assembly for the removal of a portion of the second reaction mixture according to a preferred embodiment of the present invention.

Fig. 9 is a cross-sectional side view of the filtration device shown in Fig. 8 further inserted into the open receptacle.

Fig. 10 is a graph which illustrates a dose response to digoxin generated in a sequential immunometric assay performed employing the analytical reagent mixing apparatus of the present invention.

## DESCRIPTION OF THE PREFERRED EMBODIMENTS

### Analytical Reagent Assembly

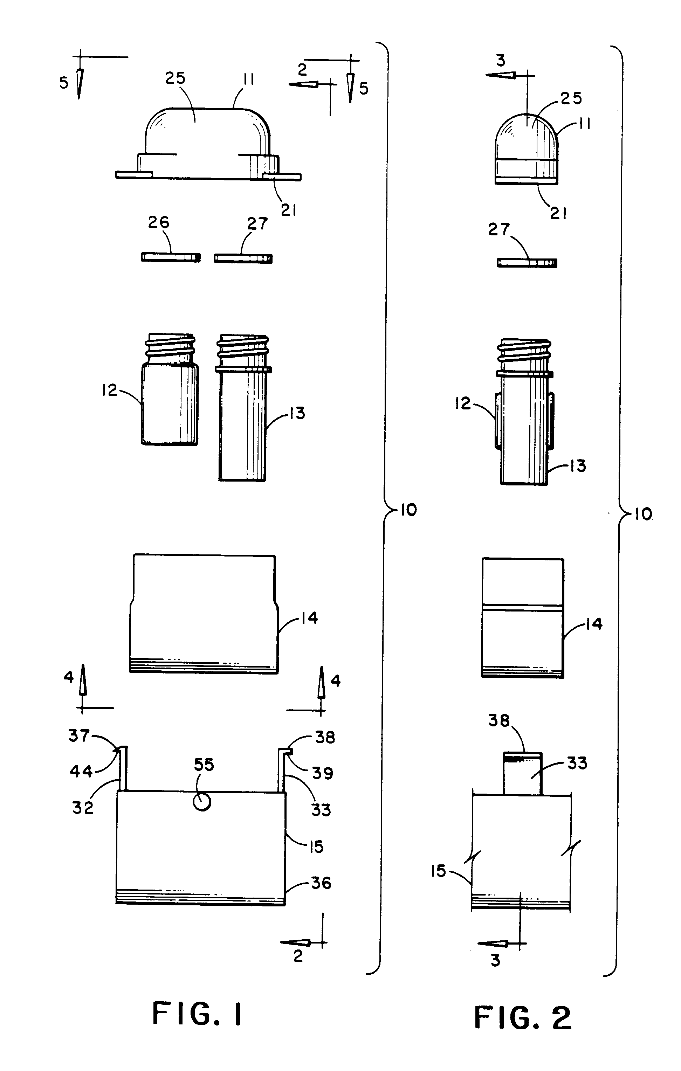

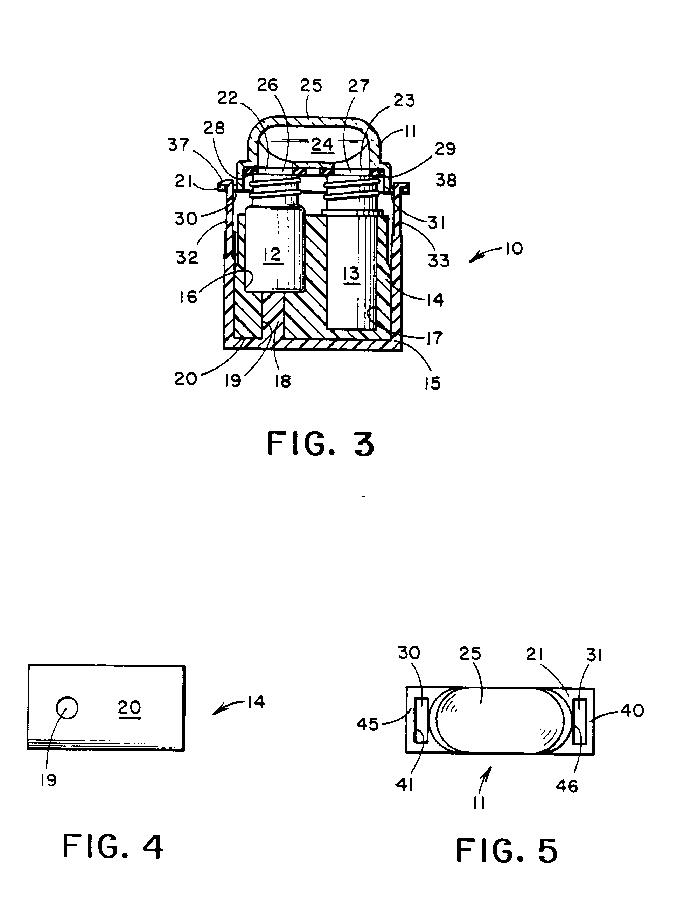

Referring now to Figs. 1 and 2, the analytical reagent assembly 10 of the present invention comprises a lid member 11, first and second receptacles 12 and 13, respectively, which are open at their upper ends and having first and second analytical reagents (not shown) disposed therein, a receptacle container 14, and a receptacle container tray 15, which can be assembled as shown in Fig. 3. Preferably, the receptacles 12 and 13 are threaded at their upper ends in order to receive threaded cap members (not shown) to prevent spillage of the analytical reagents disposed therein prior to assembling the analytical reagent assembly 10, at which time, the cap members are removed.

Referring now to Fig. 3, the receptacle container 14 includes first and second bores 16 and 17 having dimensions which are slightly larger than the dimensions of the receptacles 12 and 13, and into which the receptacles 12 and 13 are received. The receptacle container 14 is adapted to fit into and be received by the tray 15 whose internal dimensions are slightly larger than the dimensions of the container 14. The tray 15 includes a peg 18 which is mateable with a bore 19 in the bottom portion 20 of the receptacle container 14 (Fig. 4). Accordingly, since the receptacle container 14 can be properly received by the tray 15 only with the peg 18 inserted into the bore 19, the orientation of the receptacles 12 and 13 in the receptacle container 14, and therefore the respective analytical reagents disposed therein, will be assured.

The lid member 11 of the assembly 10 comprises a lower base portion 21 having first and second shouldered openings 22 and 23, and an open chamber 24 defined by an outer dome-shaped wall 25. Washers 26 and 27 are disposed into the shoulders 28 and 29 of the openings 22 and 23, and are adapted to fit over and onto the open upper ends of the receptacles 14 and 15, respectively. The openings 22 and 23 open into the open chamber 24 and are in direct communication with each other through the open chamber 24 to provide means for liquid flow between the first and second receptacles 12 and 13 when fitted thereon. As will be described in greater detail hereinafter, the analytical reagents disposed in the first and second open receptacles 12 and 13 traverse the open chamber 24 only upon the assembly 10 being sufficiently rotated by means for rotating the assembly 10.

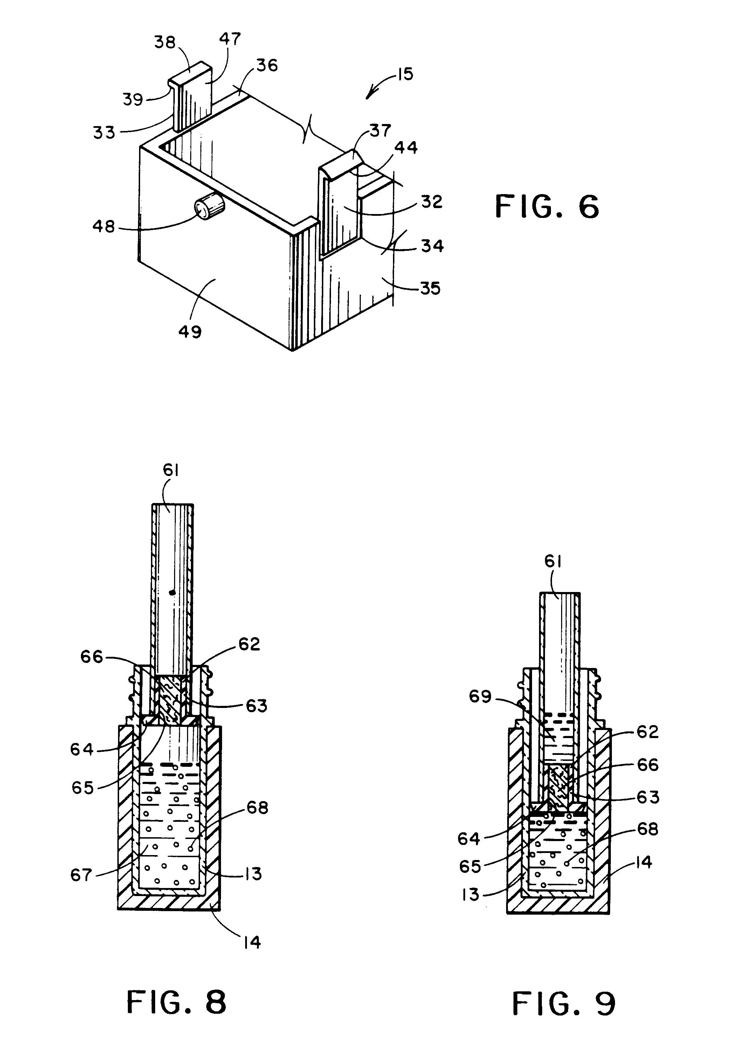

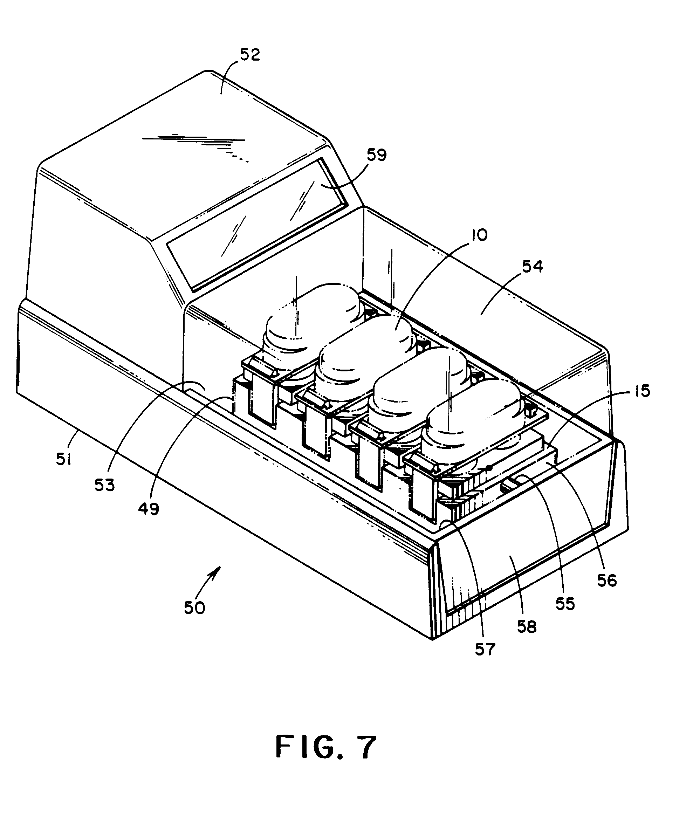

The lid member 11 further comprises first and second slots 30 and 31 (Fig. 5) located at the opposite ends of the base portion 21. The slots 30 and 31 are mateably engageable with first and second prongs 32 and 33, respectively, of the tray 15 in order to releasably secure the lid member 11 onto the first and second receptacles 12 and 13 in a fluid-tight manner. In particular (Fig. 6), the first prong 32 is mounted at its lower end to a recessed shoulder 34 located at the upper end of the side wall 35 of the tray 15 in order to provide spring-like lateral movement when pressure is applied to the outer surface thereof. Similarly, the second prong 33 is mounted at its lower end to the upper end of the side wall 36 of the tray 15 and is secured thereto so that there is little or no lateral movement thereof. The prongs 32 and 33 include flanges 37 and 38 at their upper ends, and have dimensions which are slightly smaller than, and therefore capable of being inserted through, the slots 30 and 31 of the lid member 11. It is to be understood that the distance between the slots 30 and 31 is slightly greater than the distance between the prongs 32 and 33 of the tray 15. Accordingly, in order to secure the lid member 11 onto the receptacles 12 and 13, prong 33 is first alligned with slot 31, and then flange 38 is inserted through slot 31 whereby the bottom surface 39 of flange 38 rests against the top surface 40 of the base portion 21 of the lid member 11. In order to align prong 32 with slot 30, pressure is applied to the outer surface of prong 32 with, for example, a finger, and moved inwardly until flange 37 of prong 32 is aligned with slot 30, and then inserted therethrough. When the pressure exerted onto the outer surface of prong 32 is released, prong 32 moves outwardly to return to its unaligned position with respect to slot 30 whereby the outer surface of prong 32 presses against the distal inner surface 41 of slot 30 to exert an outward pressure thereagainst, and the bottom surface 44 of flange 37 rests against the top surface 45 of the base portion 21. It is to be understood that the pressure exerted by the outer surface of prong 32 against the distal inner surface 41 of slot 30 results in the lateral movement of the lid member 11 whereby a similar pressure is exerted by the proximal inner surface 46 of slot 31 against the inner surface 47 of prong 33. Accordingly, the lid member 11 is secured onto the receptacles 12 and 13 in a fluid-tight manner. Similarly, when it is necessary to remove the lid member 11, pressure is applied to the outer surface of prong 32 to release the pressure exerted thereby and to align prong 32 with slot 30 and drawn therethrough as described above.

It is to be understood that although the analytical reagent assembly 10 has been described as set forth above, modifications can be made without departing from the teachings of the present invention. For example, one or both of the receptacles 12 and 13 can be molded into the receptacle container 14 as integral components thereof. Alternatively, the tray 15 can be adapted to receive the receptacles 12 and 13 without the use of the receptacle container 14, whereby the lid member 11 is secured to the prongs 32 and 33 of the tray 15 with the receptacles 12 and 13 positioned therebetween as described above. Furthermore, the shape and dimensions of the receptacles 12 and 13, as well as the reciprocating shape and dimensions of bores 16 and 17 in the receptacle container 14, can vary, and are not necessarily limited to the shape and dimensions thereof as shown. Still further, the tray 15 can be adapted to accomodate more than one of the analytical reagent assembly 10 (Fig. 7) in order to permit the simultaneous performance of analytical reactions involving a number of liquid test samples, as will be described in greater detail hereinafter. Alternatively, where more than two analytical reagents are necessary to perform a particular analytical reaction, the analytical reagent assembly 10 can be modified to accomodate additional reagent receptacles whereby the lid member 11 thereof is adapted to engage with and provide open communication with and between such additional reagent receptacles and the receptacles 12 and 13 as described above.

Means for Rotating

According to the present invention, the manipulation of the analytical reagents disposed in the receptacles 12 and 13 is accomplished with means for rotating the analytical reagent assembly 10 whereby depending upon the degree of rotation of the analytical reagent assembly 10 relative to vertical, the analytical reagents can either be maintained and mixed separately in the receptacles 12 and 13, or transferred and mixed between the receptacles 12 and 13 through the open chamber 24 of the lid member 11. In particular, the means for rotating the analytical reagent assembly 10 rotates the assembly 10 in a plane defined by the longitudinal axes of the receptacles 12 and 13 and about an axis of rotation defined by a point which intersects the plane. Accordingly, in order to maintain the analytical reagents in their respective receptacles 12 and 13 and prevent the premature mixing thereof, the assembly 10 is rotated about the axis of rotation less than about 90° relative to vertical, preferably about 60° relative to vertical. Alternatively, when it is desired to cause the first or second of the analytical reagents to flow through the open chamber 24 into the one or the other of the receptacles 12 or 13 and thereby result in a mixture thereof, the assembly 10 is rotated greater than about 90° relative to vertical, preferably greater than about 180°, more preferably rotated 360° relative to vertical.

Preferably, when it is desired to maintain and mix separately the analytical reagents as described above, the analytical reagent assembly 10 is rotated in alternating clockwise and counterclockwise directions to thereby oscillate the analytical reagent assembly less than about 90° relative to vertical in each direction. Similarly, when it is desired to transfer and mix the analytical reagents as described above, the analytical reagent assembly 10 is oscillated greater than about 90° relative to vertical in each direction, preferably oscillated for one or more predetermined number of revolutions, i.e., 360°, in each direction.

It is to be understood that the degree of rotation of the analytical reagent assembly with respect to accomplishing the desired manupulation of the analytical reagents and liquid test sample as described above will of course depend upon the volume thereof, as well as the internal dimensions or liquid capacity of the receptacles 12 and 13. Accordingly, the relative degree of rotation of the analytical reagent assembly 10 is not intended to be limited to the degrees of rotation as described herein, but can be readily determined by one skilled in the art apprised of the foregoing considerations without departing from the teachings of the present invention.

According to the present invention, the means for rotating the analytical reagent assembly 10 comprises a drive mechanism such as, for example, a stepping motor and a drive shaft, which is capable of performing the rotating movements as heretofore described. In particular, such drive mechanism (not shown) is coupled to a shaft member 48 situated on the proximal side wall 49 of the tray 15 (Fig. 6), such as through the drive shaft of the drive mechanism. The axis of the shaft member 48 intersects the plane defined by the longitudinal axes of the receptacles 12 and 13 to provide the axis of rotation as described above about which the analytical reagent assembly 10 is rotated. It is to be understood that the axis of rotation provided by the shaft member 48 is not limited to the position or location of the shaft member 48 on the tray 15 as shown. In particular, the point of intersection of the plane, i.e., the axis of rotation, can be at any point along the plane defined by the longitudinal axes of the receptacles 12 and 13 provided, of course, that the desired rotation of the analytical reagent assembly 10 can nevertheless be achieved to cause the mixing and transfer of the analytical reagents between the receptacles 12 and 13 as described above.

The rotating movements of the assembly 10 can be controlled by timing means (not shown), such as a programmed computer, microprocessor, and the like, for driving the drive mechanism at a predetermined speed, preferably from between about 10 and about 45 r.p.m., more preferably from between about 15 r.p.m. and about 30 r.p.m., as well as for controlling the direction and timing of the drive mechanism operation. As will be understood by one skilled in the art, such predetermined speed is not intended to be limited as described herein, and will depend upon the requirements of the particular analytical reaction being performed. Preferably, the drive motor is capable of reversing and stopping at predetermined positions, and can be monitored by a sensor (not shown) such as that known in the art to detect the rotational positioning of the drive motor or drive shaft. The sensor provides information to the control circuity of the timing means for the purpose of determining the home position of the drive mechanism, i.e., upright position of the assembly 10 as shown in Fig. 3, and the degree of rotation of the assembly 10 relative to the home position, and to determine if any rotating position misalignment has occurred during the operational cycle. Accordingly, depending upon the particular analytical reaction being performed, the drive mechanism and timing means are capable of providing the necessary number and direction of rotating movements of the assembly 10 over a predetermined period of time, as well as periods of nonmovement where one or more stationary positions of the assembly 10 is desired in order to incubate the analytical reagents.

Referring now to Fig. 7, the analytical reagent mixing apparatus 50 of the present invention preferably comprises the analytical reagent assembly 10, the drive mechanism, and the timing means encased in a protective housing 51. In particular, the drive mechanism and the timing means are situated within a body portion 52 of the housing 51, and the analytical reagent assembly 10 is situated within an access area 53 of the housing 51 beneath an access lid 54. Preferably, the access lid 54 can be transparent and can be electro-mechanically controlled to provide for the automatic locking thereof during the operation of the apparatus 50. It is to be understood that the tray 15 can be modified as shown in order to simultaneously perform, independently, analytical reactions on a number of liquid test samples. For example, where it is desired to simultaneously perform analytical reactions on four liquid test samples, the analytical reagent assembly 10 comprises the tray 15 having four sets of prongs 32 and 33, four lid members 11, four sets of receptacles 12 and 13, and four receptacle holders 14 assembled as described above. The tray 15 further includes a second shaft member 55 situated on the distal side wall 56 of the tray 15, and located on the same axis of rotation of the shaft member 48 situated on the proximal side wall 49 (Fig. 6) of the tray 6, which is rotatably mounted to the inner surface 57 of the side wall 58 of the housing 51.

The housing 51 can further include a control panel 59 which can be employed to accomodate, for example, switches and indicator lights for controlling and monitoring the operation of the analytical reagent mixing apparatus 50.

Method

The analytical reagent mixing apparatus 50 of the present invention is useful for performing sequential analytical reactions between an analyte in a liquid test sample and analytical reagents, particularly immunoassays, whereby a detectable signal is provided which is measured and correlated to the amount of analyte present in the liquid test sample. In particular, once the liquid test sample is added to the first receptacle 12 of the analytical reagent assembly 10 as described above, the analytical reagent mixing apparatus 50 of the present invention performs the desired mixing and incubation steps as heretofore described. Accordingly, once the liquid test sample has been added and the lid member 11 secured as described above, the only additional step thereafter is the measurement of the detectable signal provided by the second liquid reaction mixture according to methods known in the art. Such measurement can take place with the reaction mixture remaining in a receptacle or by removing all or at least a portion of the mixture and performing the measurement outside of the reagent assembly. It is to be understood that the periods of rotation and incubation, including the ordered sequence and timing thereof, can be modified in order to perform a desired analytical reaction which requires a particular order and/or timing of, for example, various mixing and incubation steps.

According to a preferred embodiment of the present invention, the analytical reagent mixing apparatus of the present invention is particularly useful for performing a sequential immunometric assay involving binding among the analyte, a labeled reagent comprising an anti-analyte antibody reagent labeled with a detectable chemical group, and an immobilized form of the analyte or a binding analog thereof. According to such assay, the amount of labeled antibody reagent bound to the analyte from the liquid test sample or to that which is bound to the immobilized form of the analyte is determined and related to the amount of analyte present in the test sample.

The antibody component of the antibody reagent can be a whole antibody, such as any of the classes and subclasses of known immunoglobulins, e.g., IgG, IgM, and the like, or monovalent and divalent antibody fragments of IgG, conventionally known as Fab and Fab', and $F(ab')_2$, respectively. Preferably, the antibody will commonly be a divalent antibody fragment $[F(ab')_2]$ or, more preferably, a monovalent antibody fragment (Fab or Fab'). Divalent and monovalent IgG antibody fragments can be obtained according to methods known in the art employing standard proteolytic digestion procedures with pepsin or papain.

The detectable chemical group of the labeled reagent can be any material having a detectable physical or chemical property. Such materials have been well-developed in the field of immunoassays and in general any label useful in such methods can be applied to the present invention. For example, such chemical groups having detectable physical properties are those groups which are detected on the basis of their own physical properties which do not require a chemical reaction or interaction with another chemical or substance to provide a detectable signal, such as fluorescers, phosphorescent molecules, chromophores, radioisotopes, spin labels, or electroactive moieties. Chemical groups having detectable chemical properties are those groups which are detected on the basis of their own chemical reactivity or interaction with a detectant component therefor to provide a detectable signal. Such chemical groups having detectable chemical properties do not generate a detectable product or otherwise provide a detectable signal prior to interacting with such detectant component, and include enzymatically active groups such as enzymes, enzyme substrates, coenzymes, enzyme inhibitors and activators, chemiluminescent species, chemical catalysts, metal catalysts, members of enzyme channeling, fluorophor-quencher, or energy transfer pairs, and specifically bindable ligands such as biotin or a hapten.

The immobilized form of the analyte or binding analog thereof is a separable, preferably filterable, species comprising the analyte or a binding analog thereof immobilized to a solid support, such as an insoluble particle, according to methods known in the art. Useful insoluble particles are generally in the form of a bead or microsphere, although other configurations can be employed which are capable of being separated from any of the labeled reagent bound to the analyte from the liquid test sample as described above.

In carrying out such immunometric assay employing the analytical reagent mixing apparatus 50 of the present invention, the first open receptacle 12 is provided with the labeled reagent and the second open receptacle 13 is provided with the immobilized form of the analyte, either in a liquid form or a dry form, e.g., lyophilized. A first liquid immunoassay reaction mixture is formed in the first open receptacle 12 while in a stationary, substantially upright, position, by adding the liquid test sample thereto, and then the lid member 11 is engaged with the first and second open receptacles 12 and 13 as described above. The analytical reagent assembly 10 is then rotated, preferably in alternating clockwise and counterclockwise directions, for from between about 5 seconds and about 300 seconds, preferably from between about 30 seconds and about 180 seconds, in order to maintain and separately mix the analyte and the labeled reagent in the first receptacle 12, and then incubated at a stationary, substantially upright, position for from between about 5 seconds and about 600 seconds, preferably from between about 180 seconds and about 420 seconds. The assembly 10 is then rotated, preferably in alternating clockwise and counterclockwise directions, for from between about 5 seconds and about 600 seconds, preferably from between about 180 seconds and about 420 seconds, whereby the first reaction mixture traverses the open chamber 24 of the lid member 11 and flows into the second receptacle 13 containing the immobilized form of the analyte to form a second liquid reaction mixture thereof. The second reaction mixture is then mixed between the first and second receptacle 12 and 13 through the open chamber 24 upon the subsequent rotational movements of the assembly 10.

It is to be understood that the periods of time during which the first and second reaction mixtures are rotated and incubated, will vary depending upon the nature and/or assay requirements of the particular analytical reagents employed, such as, for example, the binding kinetics of a particular antibody and analyte employed in an immunoassay, and are therefore not intended to be limited to the periods of time as described above. For example, when performing an immunometric assay as described above, and as will be understood by one skilled in the art, the analyte and the labeled reagent, i.e., the first liquid reaction mixture, are mixed and incubated for a sufficient period of time in order to permit binding of substantially all of the analyte present to the labeled reagent. Similarly, the first liquid reaction mixture and the immobilized form of the analyte, i.e., the second liquid reaction mixture comprising the analyte bound to the labeled reagent, free or unbound labeled reagent, and the immobilized form of the analyte, are mixed for a sufficient period of time to permit binding of substantially all of the free labeled reagent to the immobilized form of the analyte to result in separable free and bound species, respectively, thereof.

Once the assembly 10 has been rotated for the desired period of time as described above, the rotation thereof is stopped at a substantially upright position. The lid member 11 is then disengaged, and at least a portion of the second liquid reaction mixture is removed and the detectable signal from either the free species or the bound species of the labeled reagent is measured and correlated to the amount of analyte present in the liquid test sample according to methods known in the art. For example, where the density of the particle of the immobilized form of the analyte is substantially greater than the density of the liquid portion of the second liquid reaction mixture, the immobilized form of the analyte will settle out of the mixture to provide a supernatant comprising the bound species of the labeled reagent. Alternatively, the second liquid reaction mixture, or at least a portion thereof, can be removed and centrifuged to provide a supernatant comprising the bound species of the labeled reagent.

Preferably, the bound species of the labeled reagent is separated from the free species of the labeled reagent employing a filtration device (Figs. 8 and 9) comprising a hollow, cylindrical tube member 61 and a rubber piston or plug member 62 which is fitted into the proximal opening of the tube member 61. The plug member 62 comprises a body 63 having a slightly larger outer diameter than the inner diameter of the tube member 61 whereby a fluid-tight seal is created when inserted therein, and an annular, radially extending lip or flange 64 at the proximal end thereof. The outer diameter of the flange 64 is slightly larger than the inner diameter of the receptacle 13 containing the second reaction mixture in order to provide a fluid-tight seal when inserted therein. The plug member 62 includes an axial bore 65 which opens at both the proximal and distal ends of the plug member 62, and into which is press-fitted an elongate, cylindrical, porous, filter member 66 having an outer diameter which is slightly larger than the inner diameter of the axial bore 67.

7

The porous filter member 66 is substantially impervious to the immobilized form of the analyte and permeable to the liquid portion and the bound species of the second reaction mixture. Preferably, the filter member 66 comprises a non-fibrous porous material made from, for example, a synthetic resin such as polyethylene, polypropylene, or polyvinylidene, and having a plurality of interconnected voids defining passageways or omnidirectional, interconnecting pores therethrough.

In particular, the bound and free species are separated from each other employing the filtration device by inserting the proximal end thereof into the open receptacle 13 containing the second liquid reaction mixture 67 (Fig. 8), and passing the filter member 66 through the second reaction mixture 67 to the lower end of the receptacle 13 (Fig. 9). Accordingly, the free species bound to the immobilized form of the analyte 68 is trapped between the lower end of the receptacle 13 and the proximal end of the filter member 66, and a filtrate 69 comprising the bound species of the labeled reagent and the liquid portion of the second liquid reaction mixture is provided within the tube member 61. The filtrate 69, or at least a portion thereof, is removed, such as with a pipette, and the detectable signal provided by the detectable chemical group of the labeled reagent of the bound species is measured and correlated to the amount of analyte present in the liquid test sample.

It is to be understood that depending upon the final direction of the rotating movement of the analytical reagent assembly 10, the second liquid reaction mixture will finally be contained in either the first receptacle 12 or the second receptacle 13. Accordingly, where the analytical reagent assembly 10 is finally rotated whereby the second liquid reaction mixture is instead finally contained in and removed from the first receptacle 12, the outer diameter of the flange 64 is similarly larger than the inner diameter of the first receptacle 12 to provide a fluid-tight seal when inserted therein as described above.

The detectable chemical group of the bound species can be detected and measured according to methods known in the art and will depend upon the particular detectable chemical group employed. Preferably, in carrying out the method of the present invention, the detectable chemical group of the labeled reagent is an enzyme, and an enzyme-substrate indicator compound comprising, for example, a chromogen derivatized with an enzymatically-cleavable group, is employed for the detection thereof. Typically, such an enzymatically-cleavable group is cleaved by the enzyme of the labeled reagent to generate a detectable, chromogenic product which can be measured with, for example, a reflectance photometer, and correlated to the amount of analyte present in the liquid test sample.

Generally, such an enzyme-substrate compound can be added to the filtrate whereby, in the presence of the labeled reagent, the enzyme thereof interacts with the chromogenic enzyme-substrate compound to provide the detectable signal in the form of a change in color of the filtrate. Alternatively, the filter member 61 of the filtration device can be incorporated with a soluble form of the enzyme-substrate indicator compound whereby upon insertion of the filtration device into and through the second liquid reaction mixture as described above, the enzyme-substrate indicator compound is solubilized and thereby permitted to interact with any of the bound species present in the filtrate 69 to provide the detectable signal.

Preferably, the enzyme-substrate indicator compound, or any other detectant component for a particular chemical group having a detectable chemical property, is incorporated into a carrier matrix, such as a bibulous material, according to methods known in the art. Accordingly, an aliquot of the filtrate 69 is removed from the tube member 61 of the filtration device, and applied to such carrier matrix whereby the enzyme of the labeled reagent present in the filtrate 69 interacts with the enzyme-substrate indicator compound to provide the detectable signal as described above. The detectable signal can be visually observed and/or measured with an appropriate instrument known in the art, such as a reflectance photometer.

Test Kit and Apparatus

The present invention further provides a test kit comprising all of the essential elements required to conduct a desired sequential analytical reaction method employing the analytical reagent assembly of the present invention as heretofore described. The test kit is present in a commercially packaged form, and usually included with written instructions for the performance of the particular analytical reaction method.

The test kit will comprise the analytical reagent assembly 10 having the necessary analytical reagents disposed in the first and second open receptacles 12 and 13 as described above. Preferably, the test kit will include a plurality of the analytical reagent assembly 10 for carrying out the desired analytical reactions employing the analytical reagents contained therein on a plurality of liquid test samples. The analytical reagent mixing apparatus 50 comprising the means for rotating the assembly 10, and the timing means, encased in a housing 51 which is adapted to receive one or more of the analytical reagent assembly 10, can be packaged separately or together with one or more of the analytical reagent assembly 10. The analytical reagent assembly 10, including the receptacles 12 and 13, are preferably made from inexpensive materials to permit the disposal thereof after the intended use.

More preferably, the analytical reagent assembly 10 will be particularly adapted for performing immunometric assays whereby a labeled reagent, preferably an enzyme coupled to a monovalent Fab' antibody fragment, is disposed into the first reagent receptacle 12, and the analyte under determination or a binding analog thereof coupled to an insoluble particle disposed in the second reagent receptacle 13. Preferably, the test kit for performing such immunometric assays will also include indicator means such as a test strip comprising a bibulous reagent pad incorporated with an indicator for the labeled reagent, preferably a chromogen derivatized with an enzymatically cleavable group, and the filtration device as described above.

The analytical reagent assembly 10 is packaged with cap members (not shown) engaged with the first and second receptacles 12 and 13, preferably threaded, to prevent spillage thereof, and which can be readily removed. Furthermore, one of the reagent receptacles, generally the second reagent receptacle 13, can be covered or otherwise protected by a break-away seal or sheath in order to provide a protective mechanism to prevent a user of the test kit from adding the liquid test sample to the incorrect receptacle, and which can be readily removed subsequent to the addition of the liquid test sample to the correct reagent receptacle.

In particular, the present invention will now be illustrated by the following example:

EXAMPLE 1

Automated Performance of a Sequential Immunoassay for Digoxin

A sequential immunometric assay for the determination of digoxin was performed automatically employing the analytical reagent mixing apparatus 50 comprising the analytical reagent assembly 10 coupled to a stepping motor controlled by a programmed Epson HX-40 computer (Epson, Torrance, CA, USA), and the following reagents:

(a) Immobilized Digitoxigenin Reagent Particles

An immobilized form of digitoxigenin (digoxin binding analog) was prepared according to the method described in U.S. patent application Serial No. 939,902, filed December 9, 1986 (= US-A-4 822 747) and entitled "Substantially Stable Immobilized Hapten Reagent For Use In Immunometric Assays". According to such method, digitoxigenin in anhydrous pyridine was treated with 4-dimethylaminopyridine and p-nitrophenylchloroformate to obtain 3-digitoxigeninyl-p-nitrophenyl carbonate, which was then carboxy functionalized employing 6-aminocoproic acid and triethylamine in anhydrous pyridine to obtain 3-0-(5-carboxypentan-1-carbamoyl) digitoxigenin. The carboxy functionalized digitoxigenin in anhydrous N,N-dimethylformamide (DMF) was then treated with triethylamine, N-hydroxysuccinimide and N,N-dicyclohexylcarbodiimide to obtain 3-0-(5-carboxypentan-1-carbamoyl)digitoxigenin (activated digitoxigenin).

The immobilized reagent comprising the activated digitoxigenin covalently bound to the external amine groups of an aminoethyl-derivatized polyacrylamide gel particle was prepared by first forming a suspension of 2 grams of Aminoethyl BIO-GEL® P-2 resin (Bio-Rad Laboratories, Richmond, CA, USA; Lot No. 28945, 1.39 meq. amine functional groups/dry gram of resin) in 10 ml of DMF which was incubated at room temperature for 48 hours. A 0.01 ml aliquot of the supernate from the aminoethyl-derivatized BIO-GEL® P-2 resin in the DMF solution was removed and replaced with 0.01 ml of the activated digitoxigenin to form a reaction solution comprising 0.05 $\mu$moles of activated digitoxigenin per dry gram of the resin and gently mixed on a rotary mixer (end-over-end agitation) at room temperature for 48 hours, washed with a buffer solution and water, and then dried by lyophylization.

(b) Labeled Reagent

An enzyme-antibody conjugate preparation (0.3 nM in 50 mM sodium phosphate, 50 mM sodium chloride, mM magnesium chloride, 100 $\mu$g/mL bovine serum albumin, and 0.02% sodium azide, pH 7.4) comprising a single monovalent anti-digoxin antibody fragment (Fab') coupled to a single $\beta$-D-galactosidase enzyme component was employed as the labeled reagent. The monovalent antibody fragment was derived from a monoclonal antibody specific for digoxin (see, for example, Porter, *Biochem. J.*, Volume 73, p. 119 [1959] and Nisonoff, *Methods Med. Res.*, Volume 10, p. 132 [1964]) and labeled with $\beta$-D-galactosidase (see, for example, Ishikawa, *J. Biochem.*, Volume 96, p. 659 [1984]; Kato et al., *J. Immunol,* Volume 116, p.

1554 [June 1976]; and Yoshitake et al., *Euro. J. Biochem.*, Volume 101, p. 395 [1977]). The monovalent antibody fragment-$\beta$-D-galactosidase conjugate was electrophoretically purified on a polyacrylamide gel (polyacrylamide gel electrophoresis) to result in a substantially pure conjugate preparation comprising a single Fab′ component and a single $\beta$-D-galactosidase component, as described in U.S. patent application Serial No. 939,640, filed December 9, 1986 and entitled "Substantially Pure Enzyme-Antibody Monoconjugate Preparation". According to such method, a vertical slab polyacrylamide gel gradient (5%-8%T,2°C) was prepared by pouring, in series, a 5%T,2%C gel solution (40%T,2%C acrylamide) and a 8%T,2%C gel solution (40%T,2%C acrylamide and sucrose) to provide a linearly increasing concentration gradient. The $\beta$-D-galactosidase component was reacted with the antibody component to result in a reaction mixture comprising, as separately migrateable species, a free $\beta$-D-galactosidase component and conjugates comprising one or more of the $\beta$-D-galactosidase component coupled to one or more of the antibody component. The reaction mixture was applied to the gel gradient whereby the conjugates and the free $\beta$-D-galactosidase component were separated from each other into distinct concentration zones thereon and the conjugate employed in the present immunoassay isolated from its respective concentration zone.

(c) Chromogenic Enzyme Substrate Indicator Compound

The chromogenic indicator compound was prepared according to the method described in U.S. patent application Serial No. 939,855, filed December 9, 1986 (= US-A-4 810 636) and entitled "Chromogenic Acridinone Enzyme Substrates". According to such method, a 7-hydroxy-9,9-dimethyl-9H-acridin-2-one chromogen, derived from a 7-hydroxy-1,3-dichloro-9,9-dimethyl-9H-acridin-2-one intermediate, was reacted with acetobromogalactose and silver oxide in ethylacetate/quinoline to prepare a 7-(tetra-O-acetyl-$\beta$-D-galactopyranosyloxy)-9,9-dimethyl-9H-acridinone, which was then hydrolyzed with sodium methoxide in methanol to obtain the desired 7-$\beta$-D-galactopyranosyloxy-9,9-dimethyl-9H-acridin-2-one indicator compound employed herein.

Assay Method

In carrying out the assay, the enzyme-antibody conjugate preparation (750 $\mu$L) was placed in a glass vial 12 (15 mm diameter x 28 mm height) and the immobilized digitoxigenin reagent (30 mg) was placed in a plastic vial 13 (12 mm outer diameter and 8.5 mm inner diameter x 37 mm height). The glass vial 12 and the plastic vial 13 were placed in the bores 16 and 17, respectively, of the receptacle holder 14, and a first liquid reaction mixture was formed by adding 30 $\mu$L of a human serum test sample containing digoxin to the glass vial 12 containing the labeled reagent. The analytical reagent assembly 10 was assembled by placing the receptacle holder 14 into the receptacle holder tray 15, and the lid member 11 secured thereto as described above. The shaft member 48 of the receptacle holder tray 15 was coupled to the stepping motor with the analytical reagent assembly 10 and vials 12 and 13 in a stationary, upright position.

The drive motor, under the control of the programmed computer, mixed the test sample and the labeled reagent in the glass vial 12 by rotating (30 r.p.m., alternating clockwise and counterclockwise directions) the analytical reagent assembly 10 to 57° relative to vertical. After 1 minute, the rotating movements of the analytical reagent assembly 10 ceased and the analytical reagent assembly 10 was incubated at a stationary, upright position for 5 minutes. During the mixing and incubation periods, the bound species of the labeled reagent comprising digoxin bound to the labeled reagent was formed. After 5 minutes, the first liquid reaction mixture in the glass vial 12 comprising the test sample and the labeled reagent was mixed with the immobilized reagent in the plastic vial 13 by rotating the analytical reagent assembly 10 (alternating counterclockwise and clockwise directions, respectively) for 5 complete revolutions in each direction, for 4 minutes. Such alternating rotational movements permitted the first liquid reaction mixture in the glass vial 12 to traverse the open chamber 24 of the lid member 11 and into the plastic vial 13 containing the immobilized reagent to form the second liquid reaction mixture therewith. Accordingly, the continuing alternating, rotating movements mixed the second reaction mixture between the glass vial 12 and the plastic vial 13 through the open chamber 24 whereby any of the free or unbound labeled reagent, i.e., not bound to digoxin, became bound to and immobilized by the immobilized reagent. After 4 minutes, the alternating, rotating movements of the analytical reagent assembly 10 ceased after a final counterclockwise direction of rotation whereby the second liquid reaction mixture was transferred to and finally remained in the plastic vial 13.

The lid member 11 was removed and a filtration device such as previously described (25 $\mu$m porous plastic filter member 66, 9.5 mm outer diameter flange 64, Porex Technologies, Fairburn, GA, USA) was inserted into the plastic vial 13 and passed through the second liquid reaction mixture to the lower end of the plastic vial 13 to form a filtrate comprising the bound species of the labeled reagent in the tube member 61 of the filtration device. The labeled reagent bound to the immobilized reagent which was impermeable to the filter member 66, remained between the filter member 66 and the lower end of the plastic vial 13. An aliquot (30 $\mu$L) of the filtrate was removed with a pipette and applied to a reagent strip incorporated with the substrate.

The rate of color formation resulting from the interaction between the $\beta$-D-galactosidase and the acridinone-$\beta$-D-galactopyranoside was measured at 630 nm between about 60 to 80 seconds after sample application to the reagent pad, in order to determine the $\beta$-D-galactosidase activity of the conjugate labeled reagent from the filtrate, i.e., the bound species. The reactivity measurements (TABLE 1) were made on a Seralyzer® reflectance photometer (Miles Inc., Elkhart, IN, USA) attached to an HP-85 computer (Hewlett-Packard Company, Palo Alto, CA, USA) through a multiple port interface.

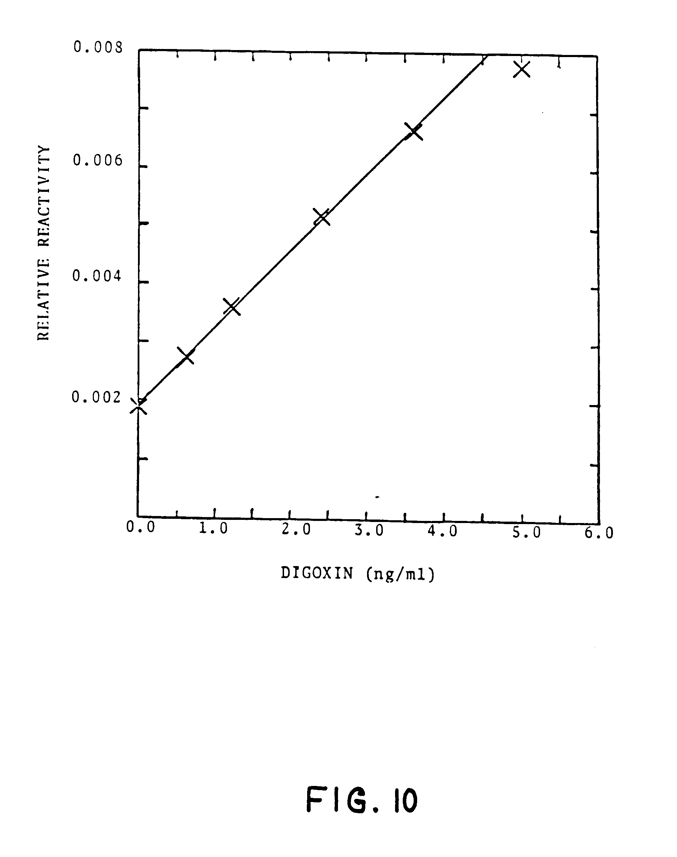

The resulting dose response to digoxin based upon the data shown in Table 1 is shown in Fig. 10.

TABLE 1

| Digoxin Concentration (ng/mL) | Reactivity x $10^3$ |
|---|---|
| 0 | 1.90 |
| 0.6 | 2.76 |
| 1.2 | 3.61 |
| 2.4 | 5.18 |
| 3.6 | 6.67 |
| 5.0 | 7.76 |

## Claims

1. An analytical reagent mixing apparatus for performing sequential analytical reactions characterized by:
   (a) an analytical reagent assembly comprising first and second open receptacles having first and second analytical reagents disposed therein and a lid member engaged with said receptacles, said lid member comprising means for providing open liquid communication between said first and second receptacles; and
   (b) means for rotating said assembly whereby a liquid added to at least one of the receptacles can be either (i) maintained and mixed separately with the reagent in said receptacle or (ii) transferred and mixed between said receptacles through said means for providing open liquid communication.

2. The apparatus of Claim 1, characterized in that the rotating means rotates said assembly in a plane defined by the longitudinal axes of said receptacles.

3. The apparatus of Claim 2, characterized in that the rotating means is capable of rotating said assembly less than 90° relative to vertical in alternating clockwise and counterclockwise directions whereby said liquid is maintained and mixed separately with the reagent in said receptacle.

4. The apparatus of Claim 2, characterized in that the rotating means is capable of rotating said assembly greater than about 90° relative to vertical in alternating clockwise and counterclockwise directions whereby said liquid is transferred and mixed between said receptacles through said means for providing open liquid communication.

5. The apparatus of Claim 4, characterized in that said means for rotating said assembly rotates said assembly 360° relative to vertical, preferably for a predetermined number of revolutions in alternating clockwise and counterclockwise directions.

6. The apparatus of any of Claims 1 to 5, characterized in that said first analytical reagent is a first immunoassay reagent comprising a labeled reagent comprising a member of a specific binding pair labeled with a detectable chemical group, preferably an anti-analyte antibody reagent labeled with a detectable chemical group, and said second analytical reagent is a second immunoassay reagent comprising an immobilized form of the other member of said specific binding pair, preferably an immobilized form of said analyte or a binding analog thereof, preferably immobilized to a water suspendable particle.

7. The apparatus of any of Claims 1 to 6, characterized by timing means for controlling the rotating movements of said assembly for predetermined periods of time, said timing means preferably cooperating with said rotating means to sequentially (i) rotate said assembly less than about 90° relative to vertical in alternating clockwise and counterclockwise directions for a predetermined period of time, (ii) maintain said assembly in a substantially upright, stationary position for a predetermined period of time, and (iii) rotate said assembly greater than about 90° relative to vertical in alternating clockwise and counterclockwise directions for a predetermined period of time.

8. A method for performing sequential analytical reactions between an analyte in a liquid test sample and analytical reagents employing the apparatus of any of Claims 1 to 7, whereby a detectable signal is provided which is measured and correlated to the amount of analyte present in the liquid test sample, said method characterized by the steps of:
    (a) forming a first liquid reaction mixture by adding said liquid test sample to said first analytical reagent in said first receptacle of said analytical reagent assembly;
    (b) engaging said lid member with said first and second receptacles of said analytical reagent assembly;
    (c) rotating said assembly for a predetermined period of time whereby said first liquid reaction mixture is maintained and mixed separately in said first receptacle and said second analytical reagent is maintained in said second receptacle;
    (d) rotating said assembly for a predetermined period of time whereby said first reaction mixture traverses said means for providing open liquid communication of said lid member to form a second liquid reaction mixture comprising said first liquid reaction mixture and said second analytical reagent; and
    (e) removing at least a portion of said second liquid reaction mixture and measuring the detectable signal provided thereby.

9. A method for performing a sequential immunometric assay for determining an analyte in a liquid test sample employing the apparatus of any of Claims 1 to 7, said assay involving binding among the analyte, an antianalyte antibody reagent labeled with a detectable chemical group, and an immobilized form of the analyte or a binding analog thereof, wherein the amount of labeled antibody reagent which does not become bound to the immobilized form of the analyte or analog is determined and related to the amount of analyte present in the test sample, said method characterized by the steps of:
    (a) forming a first liquid immunoassay reaction mixture by adding said liquid test sample to said labeled antibody reagent in said first receptacle of said analytical reagent assembly;
    (b) engaging said lid member with said first and second receptacles of said analytical reagent assembly;
    (c) rotating said assembly less than about 90° relative to vertical in a plane defined by the longitudinal axes of said receptacles for a predetermined period of time in alternating clockwise and counterclockwise directions whereby said first liquid reaction mixture is maintained and mixed separately in said first receptacle and said immobilized analyte or binding analog thereof is maintained in said second receptacle;
    (d) rotating said assembly in said plane for a predetermined number of complete revolutions in alternating clockwise and counterclockwise directions for a predetermined period of time whereby said first liquid immunoassay reaction mixture traverses said means for providing open communication of said lid member to form a second liquid immunoassay reaction mixture comprising said first liquid immunoassay reaction mixture and said immobilized form of a said analyte or analog thereof; and
    (e) removing at least a portion of said second liquid immunoassay reaction mixture comprising labeled antibody which has not become bound to the immobilized form of the analyte or analog thereof and measuring the detectable signal provided therefrom.

10. The method of Claim 9, characterized in that said immobilized form of the analyte or analog comprises a filterable particle, preferably a water suspendable particle, and wherein step (f) involves passing filter means through said second liquid immunoassay reaction mixture to separate labeled antibody that has become associated with said filterable particle from labeled reagent that remains in solution to form a filtrate thereof, removing a portion of the resulting filtrate, and measuring the detectable signal provided thereby.

**Revendications**

1. Mélangeur de réactifs pour procéder à des réactions analytiques séquentielles caractérisé par:
   (a) un ensemble à réactifs comprenant un premier et un deuxième récipients ouverts dans lesquels sont disposés respectivement un premier et un deuxième réactifs et qui sont fermés par un couvercle, ledit couvercle comprenant un dispositif de communication ouverte des liquides entre lesdits premier et deuxième récipients; et
   (b) un dispositif de rotation dudit ensemble par lequel un liquide ajouté à au moins un des récipients peut ainsi être soit (i) maintenu et mélangé séparément avec le réactif dans ledit récipient ou (ii) transféré et mélangé entre lesdits récipients par ledit dispositif de communication ouverte des liquides.

2. Le mélangeur selon la revendication 1 caractérisé en ce que le dispositif de rotation fait tourner ledit ensemble dans un plan défini par les axes longitudinaux desdits récipients.

3. Le mélangeur selon la revendication 2 caractérisé en ce que le dispositif de rotation est capable de faire tourner ledit ensemble à moins de 90° par rapport à la verticale en alternant le sens des aiguilles d'une montre et le sens contraire des aiguilles d'une montre, ledit liquide étant ainsi maintenu et mélangé séparément avec le réactif dans ledit récipient.

4. Le mélangeur selon la revendication 2 caractérisé en ce que le dispositif de rotation est capable de faire tourner ledit ensemble à plus de 90° environ par rapport à la verticale en alternant le sens des aiguilles d'une montre et le sens contraire des aiguilles d'une montre, ledit liquide étant ainsi transféré et mélangé entre lesdits récipients par ledit dispositif de communication ouverte des liquides.

5. Le mélangeur selon la revendication 4 caractérisé en ce que ledit dispositif de rotation dudit ensemble fait tourner ledit ensemble de 360° par rapport à la verticale, de préférence pendant un nombre prédéterminé de tours, en alternant le sens des aiguilles d'une montre et le sens contraire des aiguilles d'une montre.

6. Le mélangeur selon une des revendications 1 à 5 caractérisé en ce que ledit premier réactif est un premier réactif d'immunoessai comprenant un réactif marqué comportant un élément d'une paire de liaison spécifique marquée avec un radical chimique détectable, de préférence un réactif anticorps anti-analyte marqué avec un radical chimique détectable et ledit deuxième réactif analytique est un deuxième réactif d'immunoessai comprenant une forme immobilisée de l'autre élément de ladite paire de liaison spécifique, de préférence une forme immobilisée dudit analyte ou un analogue de liaison de celui-ci, de préférence immobilisé sur une particule pouvant être mise en suspension dans l'eau.

7. Le mélangeur selon une des revendications 1 à 6 caractérisé par une minuterie destinée à commander les mouvements de rotation dudit ensemble pendant des périodes de temps prédéterminées, ladite minuterie coopérant de préférence avec ledit dispositif de rotation pour, successivement, (i) faire tourner ledit ensemble à moins de 90° environ par rapport à la verticale en alternant le sens des aiguilles d'une montre et le sens contraire des aiguilles d'une montre pendant une période de temps prédéterminée, (ii) maintenir ledit ensemble dans une position stationnaire essentiellement verticale pour une période de temps prédéterminée et (iii) faire tourner ledit ensemble de plus de 90° environ par rapport à la verticale en alternant le sens des aiguilles d'une montre et le sens contraire des aiguilles d'une montre pendant une période de temps prédéterminée.

8. Une méthode pour procéder à des réactions analytiques séquentielles entre un analyte dans un échantillon de test liquide et des réactifs en utilisant le mélangeur selon une des revendications 1 à 7, un signal détectable ainsi produit étant mesuré et corrélé à la quantité d'analyte présente dans l'échantillon de test liquide, ladite méthode étant caractérisée par les étapes de:
   (a) formation d'un premier mélange réactif liquide par addition dudit échantillon de test liquide audit premier réactif dans ledit premier récipient dudit ensemble à réactifs;
   (b) fermeture par ledit couvercle dans lesdits premier et deuxième récipients dudit ensemble à

EP 0 320 752 B1

réactifs ;

(c) la rotation dudit ensemble pendant une période de temps prédéterminée, ledit premier mélange réactif liquide étant maintenu et mélangé séparément dans ledit premier récipient et ledit deuxième réactif étant maintenu dans ledit deuxième récipient;

(d) la rotation dudit ensemble pendant une période de temps prédéterminée, ledit premier mélange réactif traversant ledit dispositif de communication ouverte des liquidesdudit couvercle pour former un deuxième mélange réactif liquide comprenant ledit premier mélange réactif liquide et ledit deuxième réactif ; et

(e) l'élimination d'au moins une partie dudit deuxième mélange réactif liquide et la mesure du signal détectable ainsi produit.

**9.** Une méthode pour accomplir un essai immunométrique séquentiel pour déterminer un analyte dans un échantillon de test liquide en utilisant le mélangeur des revendications 1 à 7, ledit essai impliquant la liaison entre l'analyte, un réactif anticorps anti-analyte marqué avec un radical chimique détectable et une forme immobilisée de l'analyte ou un analogue de liaison de celui-ci, dans laquelle la quantité de réactif anticorps marqué qui n'est pas lié à la forme immobilisée de l'analyte ou de son analogue est déterminée et corrélée à la quantité d'analyte présente dans l'échantillon de test, ladite méthode étant caractérisée par les étapes de:

(a) la formation d'un premier mélange réactif d'immunoessai liquide par addition dudit échantillon de test liquide audit réactif anticorps marqué dans ledit premier récipient dudit ensemble à réactifs ;

(b) la fermeture par ledit couvercle desdits premier et deuxième récipients dudit ensemble à réactifs ;

(c) la rotation dudit ensemble de moins de 90° environ par rapport à la verticale dans un plan défini par les axes longitudinaux desdits récipients pendant une période de temps prédéterminée en alternant le sens des aiguilles d'une montre et le sens contraire des aiguilles d'une montre, ledit premier mélange réactif liquide étant maintenu et mélangé séparément dans ledit premier récipient et ledit analyte immobilisé ou l'analogue de liaison de celui-ci étant maintenu dans ledit deuxième récipient;

(d) la rotation dudit ensemble dans ledit plan pendant un nombre prédéterminé de tours complets en alternant le sens des aiguilles d'une montre et le sens contraire des aiguilles d'un montre pendant une période de temps prédéterminée, ledit premier mélange réactif liquide d'immunoessai traversant ledit dispositif de communication ouverte dudit couvercle pour former un deuxième mélange réactif liquide d'immunoessai comprenant ledit premier mélange réactif liquide d'immunoessai et ladite forme immobilisée dudit analyte ou de son analogue; et

(e) l'élimination d'au moins une partie dudit deuxième mélange réactif liquide d'immunoessai comprenant l'anticorps marqué qui ne s'est pas lié à la forme immobilisée de l'analyte ou de son analogue et la mesure du signal détectable ainsi produit.

**10.** La méthode de la revendication 9 caractérisée en ce que ladite forme immobilisée de l'analyte ou de son analogue comprend une particule filtrable, de préférence une particule pouvant être suspendue dans l'eau, et dans laquelle l'étape (f) implique le passage d'un dispositif de filtrage par ledit deuxième mélange réactif liquide d'immunoessai pour séparer l'anticorps marqué qui s'est associé à ladite particule filtrable du réactif marqué qui reste en solution pour former un filtrat, l'élimination d'une partie du filtrat obtenu et la mesure du signal détectable ainsi produit.

**Patentansprüche**

**1.** Analysereagensmischungsvorrichtung zur Durchführung aufeinanderfolgender Analysereaktionen, **gekennzeichnet,** durch

(a) ein Analysereagensgerät, umfassend erste und zweite offene Behältnisse mit ersten und zweiten darin enthaltenen Analysereagentien, sowie einen Deckelteil, verbunden mit den genannten Behältnissen, wobei der genannte Deckelteil Elemente zur Bereitstellung einer offenen flüssigen Verbindung zwischen den genannten ersten und zweiten Behältnissen umfaßt; und

(b) ein Element zur Drehung des genannten Geräts, wodurch eine Flüssigkeit, die mindestens einem der Behältnisse zugeführt wird, entweder (i) mit dem Reagens im genannten Behältnis gehalten und gesondert vermischt oder (ii) zwischen den genannten Behältnissen durch das genannte Element zur Bereitstellung einer offenen flüssigen Verbindung hindurch übertragen und vermischt werden kann.

14

EP 0 320 752 B1

**2.** Vorrichtung nach Anspruch 1,
dadurch **gekennzeichnet,** daß
das Drehelement das genannte Gerät in einer Ebene dreht, die durch die Längsachsen der genannten Behältnisse festgelegt ist.

**3.** Vorrichtung gemäß Anspruch 2,
dadurch **gekennzeichnet,** daß
das Drehelement dazu befähigt ist, das genannte Gerät um weniger als 90° relativ zur Senkrechten in abwechselnden Richtungen im und gegen den Uhrzeigersinn zu drehen, wodurch die genannte Flüssigkeit mit dem Reagens im genannten Behältnis gehalten und gesondert vermischt wird.

**4.** Vorrichtung gemäß Anspruch 2,
dadurch **gekennzeichnet,** daß
das Drehelement dazu befähigt ist, das genannte Gerät um mehr als ca. 90° relativ zur Senkrechten in abwechselnden Richtungen in und gegen den Uhrzeigersinn zu drehen, wodurch die genannte Flüssigkeit zwischen den genannten Behältnissen durch das genannte Element zur Berteitstellung einer offenen und flüssigen Verbindung hindurch übertragen und vermischt wird.

**5.** Vorrichtung gemäß Anspruch 4,
dadurch **gekennzeichnet,** daß
das genannte Drehelement zur Drehung des genannten Geräts das genannte Gerät um 360° relativ zur Senkrechten, vorzugsweise für eine vorbestimmte Anzahl von Umdrehungen in abwechselnden Richtungen im und gegen den Uhrzeigersinn, dreht.

**6.** Vorrichtung gemäß jedem der Ansprüche 1 bis 5,
dadurch **gekennzeichnet,** daß
das genannte erste Analysereagens ein erstes Immunoreagens ist, enthaltend ein markiertes Reagens, enthaltend ein Glied eines spezifischen Bindungspaares, das mit einer nachweisbaren chemischen Gruppe markiert ist, vorzugsweise ein mit einer nachweisbaren chemischen Gruppe markiertes Anti-Analyt-Antikröperreagens, und daß das genannte zweite Analysereagens ein zweites Immunoassayreagens ist, enthaltend eine immobilisierte Form eines anderen Glieds des genannten spezifischen Bindungspaares, vorzugsweise eine immobilisierte Form des genannten Analyt oder eines Bindungsanalogs davon, vorzugsweise immobilisiert an einem in Wasser suspendierbaren Teilchen.

**7.** Vorrichtung gemäß jedem Anspruch 1 bis 6,
**gekennzeichnet** durch
ein Zeitgebungselement zur Steuerung der Drehbewegungen des genannten Geräts über vorbestimmte Zeiträume, wobei das genannte Zeitgebungselement vorzugsweise mit genanntem Drehelement zusammenwirkt, um (i) das genannte Gerät um weniger als ca. 90° relativ zur Senkrechte in abwechselnden Richtungen im und gegen den Uhrzeigersinn einen vorbestimmten Zeitraum lang aufeinanderfolgend zu drehen, (ii) das genannte Gerät in einer im wesentlichen aufrechten, stationären Stellung einen vorbestimmten Zeitraum lang zu halten und (iii) das genannte Gerät um mehr als ca. 90° relativ zur Senkrechten in abwechselnden Richtungen im und gegen den Uhrzeigersinn einen vorbestimmten Zeitraum lang zu drehen.

**8.** Verfahren zur Durchführung aufeinanderfolgender Analysereaktionen zwischen einem Analyt in einer flüssigen Testprobe und Analysereagentien, wobei man die Vorrichtung eines jeden der Ansprüche 1 bis 7 verwendet, wobei ein nachweisbaren Signal geliefert wird, das gemessen und mit der Menge an in der flüssigen Testprobe vorliegendem Analyt korreliert wird, wobei das genannte Verfahren durch die folgenden Stufen **gekennzeichnet** ist, wobei man:
(a) eine erste flüssige Reaktionsmischung bildet, indem man die genannte flüssige Testprobe dem genannten ersten Analytreagens im genannten ersten Behältnis des genannten Analysereagensgerätes zufügt;
(b) den genannten Deckelteil mit den genannten ersten und zweiten Behältnissen des genannten Analysereagensgeräts verbindet;
(c) das genannte Gerät einen vorbestimmten Zeitraum lang dreht, wodurch die genannte erste flüssige Reaktionsmischung im genannten ersten Behältnis gehalten und gesondert vermischt und das genannte zweite Analysereagens im genannten zweiten Behältnis gehalten werden;

15

(d) das genannte Gerät einen vorbestimmten Zeitraum lang dreht, wodurch die genannte erste Reaktionsmischung das genannte Element zur Bereitstellung einer offenen flüssigen Verbindung des genannten Deckelteils durchquert, um eine zweite flüssige Reaktionsmischung zu bilden, enthaltend genannte erste flüssige Reaktionsmischungen und genanntes zweites Analysereagens; und

(e) mindestens einen Anteil der genannten zweiten flüssigen Reaktionsmischung herausnimmt und das gelieferte nachweisbare Signal mißt.

9. Verfahren zur Durchführung eines aufeinanderfolgenden immunometrischen Assays zur Bestimmung eines Analyt in einer flüssigen Testprobe, wobei man die Vorrichtung eines jeden Anspruchs 1 bis 7 verwendet, wobei der genannte Assay eine Bindung zwischen dem Analyt, einem mit einer nachweisbaren chemischen Gruppe markierten Anti-Analyt-Antikörperreagens und einer immobilisierten Form des Analyt oder eines Bindungsanalogs davon einschließt, wobei die Menge an markiertem Antikörperreagens, das nicht an die immobilisierte Form des Analyt oder Analogs gebunden wird, bestimmt und zur Menge an in der flüssigen Testprobe vorliegendem Analyt in Beziehung gesetzt wird, wobei das genannte Verfahren durch die folgenden Stufen **gekennzeichnet** ist, wobei man:

(a) eine erste flüssige Immunoassayreaktionsmischung bildet, indem man die genannte flüssige Testprobe dem genannten markierten Antikörperreagens im genannten ersten Behältnis des genannten Analysereagensgerätes zufügt;

(b) den genannten Deckelteil mit den genannten ersten und zweiten Behältnissen des genannten Analysereagensgerätes verbindet;

(c) das genannte Gerät um weniger als ca. 90 ° relativ zur Senkrechten in einer Ebene, die durch die Längsachse der genannten Behältnisse festgelegt ist, einen vorbestimmten Zeitraum lang in abwechselnden Richtungen im und gegen den Uhrzeigersinn dreht, wodurch genannte erste flüssige Reaktionsmischung im genannten ersten Behältnis gehalten und gesondert vermischt und genannter immobilisierter Analyt oder dessen Bindungsanalog in genanntem zweiten Behältnis gehalten werden;

(d) genannte Vorrichtung in genannter Ebene für eine vorbestimmte Anzahl von vollständigen Umdrehungen in abwechselnden Richtungen im und gegen den Uhrzeigersinn einen vorbestimmten Zeitraum lang dreht, wodurch genannte erste flüssige Immunoassayreaktionsmischung das genannte Element zur Bereitstellung einer offenen Verbindung des genannten Deckelteils durchquert, um eine zweite flüssige Immunoassayreaktionsmischung zu bilden, enthaltend genannte erste flüssige Immunoassayreaktionsmischung und genannte immobilisierte Form eines genannten Analyt oder dessen Analogs; und

(e) mindestens einen Anteil der genannten zweiten flüssigen Immunoassayreaktionsmischung, enthaltend den markierten Antikörper, der nicht an die immobilisierte Form des Analyt oder dessen Analog gebunden worden ist, herausnimmt und das davon gelieferte nachweisbare Signal misst.

10. Verfahren gemäß Anspruch 9,
dadurch **gekennzeichnet,** daß
genannte immobilisierte Form des Analyt oder Analogs ein filtrierbares Teilchen enthält, vorzugsweise ein in Wasser suspendierbares Teilchen, und wobei Stufe (f) beinhaltet, daß man ein Filterelement durch genannte zweite flüssige Immunoassayreaktionsmischung führt, um markierten Antikörper, der mit genanntem filtrierbaren Teilchen assoziiert worden ist, von markiertem Reagens abzutrennen, das in Lösung verbleibt, um ein Filtrat davon zu bilden, einen Anteil des sich ergebenden Filtrats herausnimmt und das dabei gelieferte nachweisbare Signal misst.

16

FIG. I

FIG. 2

FIG. 3

FIG. 4

FIG. 5

FIG. 6

FIG. 8

FIG. 9

FIG. 7

FIG. 10